# EUROPÄISCHE PATENTSCHRIFT

(11) **EP 4 472 521 B1**
(45) Veröffentlichungstag und Bekanntmachung des Hinweises auf die Patenterteilung: **17.12.2025**
(21) Anmeldenummer: 23703690.0
(22) Anmeldetag: 27.01.2023
(51) Int. Cl.: A61B 10/02, A61B 17/00, A61B 17/32

(54) **GEWEBEAUFFANGSYSTEM**
TISSUE RECOVERY SYSTEM
SYSTÈME DE RÉCUPÉRATION DE TISSU

(30) Priorität: 01.02.2022 DE 102022201057
(43) Veröffentlichungstag der Anmeldung: 11.12.2024
(73) Patentinhaber: Richard Wolf GmbH, 75438 Knittlingen (DE)
(72) Erfinder: STEINHAUSER, Luis, 75438 Knittlingen (DE); TEWES, Moritz, 75015 Bretten (DE); KNOTH, Stefan, 75438 Knittlingen (DE); LAMPERT, Alexander, 75248 Ölbronn-Dürrn (DE)
(74) Vertreter: Patentanwälte Hemmer Lindfeld Frese Partnerschaft mbB
(86) Internationale Anmeldenummer: PCT/DE2023/200022
(87) Internationale Veröffentlichungsnummer: WO 2023/147827

(56) Entgegenhaltungen:
- CN-A- 107 669 300
- CN-U- 203 208 051
- US-A- 4 870 975
- US-A- 5 575 293
- US-A- 5 713 879
- US-A- 5 772 644

## Beschreibung

Die Erfindung betrifft ein Gewebeauffangsystem mit den im Anspruch 1 angegebenen Merkmalen.

Bei bestimmten operativen Behandlungsverfahren werden Gewebeabschnitte mechanisch von einem Organ entfernt und aus dem Körper herausgeführt. Beispielsweise bei einer urologischen Prostataresektion werden mittels Laser oder Hochfrequenzschneidgerät Teile der Prostata herausgelöst und in die Blase gestoßen. Um die abgetrennten Teile aus der Blase entfernen zu können, kann ein Morcellationssystem verwendet werden, bei dem durch einen Unterdruck Gewebeteile in einen Morcellator gesaugt werden. Der Morcellator weist eine Anordnung aus einem rohrartigen Innenmesser, das in einem rohrartigen Au-ßenmesser steckt, auf. Mittels des Unterdrucks wird das Gewebe am Messer gehalten und durch die Rotation des Messers wird es verkleinert. Der Unterdruck saugt dann die Gewebepartikel mit etwas Spülflüssigkeit ab. Es ist sinnvoll, das Gewebe nach der Operation pathologisch zu untersuchen, sodass es hierzu aufgefangen und von der Spülflüssigkeit separiert werden muss.

Bekannte Absaugsysteme zur Benutzung bei einem solchen Vorgang verwenden einen Vakuumbehälter in Kombination mit einer Vakuumpumpe. Die Vakuumpumpe evakuiert den Vakuumbehälter, an dem ein Auffangbehälter angeordnet ist und mit einem Schlauch verbunden ist, der zu dem Morcellator führt. Die an dem Morcellator angesaugte Spülflüssigkeit mit Gewebepartikeln fließt durch den Auffangbehälter, das Gewebe verbleibt dort und die Spülflüssigkeit fließt in den Vakuumbehälter ab. Damit der Unterdruck stets auch an dem Morcellator anliegt, muss ein entsprechender Pfad zu dem Vakuumbehälter unverschlossen bleiben. Ein Verschluss könnte sich durch eine zu große Menge an Gewebe im Auffangbehälter oder durch ein Kollabieren des Schlauches unter dem Druck der Atmosphäre aufgrund zu dünner Seitenwände ergeben. Nach der Operation wird das Vakuumsystem belüftet und der Auffangbehälter mit Gewebe entnommen. Die US 5,575,293 A beschreibt beispielsweise ein Gewebeauffangsystem mit starrem Vakuumbehälter, nach dem Oberbegriff des Anspruchs 1.

Bekannt ist eine Variante des Auffangbehälters, bei dem ein Gitterkörbchen in einem Auffangbehälter sitzt. Ebenfalls bekannt ist ein starrer Auffangbehälter, in dem ein Filterbeutel platziert ist, um Gewebe und Knochensplitter zurückzuhalten. Üblicherweise werden Auffangbehälter mit einem Fassungsvermögen für Gewebe von höchstens etwa 180ml bereitgestellt, was für eine Prostataoperation jedoch nicht immer ausreichend ist. Ist dieses Fassungsvermögen erreicht, muss die Operation unterbrochen werden, um das Vakuumsystem zu belüften, den Gewebeauffangbehälter zu entnehmen und zu entleeren. Anschließend wird das Morcellationssystem erneut evakuiert. Eine steife Ausbildung des Auffangbehälters ist notwendig, damit er unter Vakuum nicht den Pfad der Flüssigkeit verschließt. Damit entspricht das Transportvolumen des Behälters zwingend mindestens dem Fassungsvermögen. Dies und der erhöhte Aufwand für die Sterilisation führt zu hohen Gesamtkosten.

Vor diesem Hintergrund liegt der Erfindung die Aufgabe zugrunde, ein verbessertes Gewebeauffangsystem vorzuschlagen, das kostengünstig herstellbar ist, eine rasche und einfache Sterilisation erlaubt und ein geringeres Lager- oder Transportvolumen erfordert.

Diese Aufgabe wird gemäß der Erfindung durch ein Gewebeauffangsystem mit den in Anspruch 1 angegebenen Merkmalen gelöst. Vorteilhafte Ausgestaltungen der Erfindung sind in den Unteransprüchen, der nachfolgenden Beschreibung und den Zeichnungen angegeben.

Das erfindungsgemäße Gewebeauffangsystem weist eine Pumpe und einen Gewebeauffangbehälter zum Abtrennen von Gewebepartikeln aus einer Flüssigkeit auf. Erfindungsgemäß ist vorgesehen, dass der Gewebeauffangbehälter einen biegeweichen Innenbeutel und einen biegeweichen Außenbeutel aufweist, der Innenbeutel schlaff in dem Außenbeutel angeordnet und von diesem umschlossen ist und einen Zulauf für eine Gewebepartikel aufweisende Flüssigkeit aufweist, der Innenbeutel zumindest bereichsweise eine offenmaschige Wandung aufweist, die dazu ausgebildet ist, Gewebepartikel zu fangen, und der Außenbeutel flüssigkeitsundurchlässig ist und einen Ablauf zum Abführen von Flüssigkeit aufweist. Dabei steht die Pumpe mit dem Zulauf in Fluidverbindung und ist stromaufwärts des Zulaufs angeordnet ist, um Flüssigkeit mit zumindest leichtem Überdruck durch den Zulauf in den Innenbeutel zu drücken.

Der Begriff "schlaff" bezüglich der Anordnung des Innenbeutels in dem Außenbeutel ist hier dahingehend zu verstehen, dass der Innenbeutel keine Versteifungselemente aufweist. Er ist ohne eine äußere Versteifung in dem Außenbeutel angeordnet. Der Innenbeutel kann sich daher stets frei in dem Außenbeutel bewegen und seine Form ändern. Insbesondere kann der Innenbeutel bevorzugt lediglich an seinem oberen Ende mit dem Außenbeutel verbunden sein. Dies führt dazu, dass die Anordnung aus Innenbeutel und Außenbeutel vor der Benutzung flach ist und zudem flach aufgerollt werden kann.

Der Gewebeauffangbehälter unterscheidet sich folglich deutlich von vorangehend dargestellten bekannten Ausführungen. Statt eines starren Behälters mit einem darin angeordneten Gitterkörbchen oder ähnlichem werden lediglich zwei ineinander angeordnete Beutel eingesetzt. Der Außenbeutel umgibt dabei den Innenbeutel und ist flüssigkeitsdicht gestaltet. Flüssigkeit, insbesondere eine Spülflüssigkeit, welche sich in dem Außenbeutel befindet, kann folglich ausschließlich durch den Ablauf wieder aus dem Gewebeauffangbehälter austreten. Der Ablauf könnte mit einem weiteren Behälter oder einem geeigneten Entsorgungssystem verbunden sein und die abgeführte Flüssigkeit verarbeiten.

Der Außenbeutel ist biegeweich und weist daher eine von der momentanen Füllmenge abhängige Form auf. Steigt das von dem Außenbeutel aufgenommene Volumen, passt sich die Form des Außenbeutels durch eine entsprechende Auswölbung an. Es ist denkbar, den Außenbehälter aus PVC oder einem anderen, vorzugsweise sterilisierbaren Material auszubilden. Besonders bevorzugt ist der Außenbeutel durchsichtig, um eine Sichtkontrolle des Flüssigkeitspegels und des Innenbeutels durchführen zu können.

Der Innenbehälter ist ebenso biegeweich, jedoch nicht flüssigkeitsdicht. Stattdessen weist er zumindest bereichsweise eine offenmaschige Wandung auf, durch die in den Zulauf fließende Flüssigkeit aus dem Innenbehälter nach außen, d.h. in den Außenbeutel, treten kann. Die offenmaschige Struktur der Wandung erlaubt das Zurückhalten von Gewebeteilchen, die in der Flüssigkeit enthalten sind. Durch die Anordnung des Innenbeutels vollständig innerhalb des Außenbeutels kann rundum eine offenmaschige Wandung vorliegen und die Fläche des Innenbeutels zumindest weitgehend vollständig für das Zurückhalten von Gewebepartikeln genutzt werden. Es ist vorstellbar, dass auch der Innenbeutel aus einem durchsichtigen Material ausgebildet ist. Dies ist indes nicht erforderlich, sondern auch durch eine großflächige Perforation könnte ein Füllstatus des Innenbeutels durch Sichtkontrolle geprüft werden.

Gewebeteilchen, die von der Wandung des Innenbeutels aufgefangen werden, sammeln sich in dem Innenbeutel an. Je größer die perforierte Fläche ist, desto mehr offene Maschen stehen zumindest am Anfang der Benutzung zum Durchströmen der Flüssigkeit zur Verfügung. Erst bei einer vollständigen Belegung aller Maschen, die erst bei weitgehend vollständiger Befüllung des Innenbeutels zu erwarten ist, würde es zum Verschließen des Innenbeutels und damit zu einer erschöpften Aufnahmefähigkeit führen. Durch stetig nachströmende Flüssigkeit mit darin enthaltenen Gewebepartikeln könnten zumindest in einem dem Zulauf nahen Bereich Verwirbelungen an der Innenseite des Innenbeutels auftreten, die zugesetzte Maschen zumindest zeitweise wieder freilegen. Wird die Flüssigkeit kontinuierlich aus dem Außenbeutel abgelassen, ist der Flüssigkeitspegel in dem Außenbeutel begrenzt. Der Innenbeutel wird dann höchstens teilweise unterhalb des Flüssigkeitspegels ragen, sodass Gewebepartikel dann nicht aufschwimmen und dabei die offenmaschige Wandung zusetzen.

Die offenmaschige Wandung ist als ein flächiges, biegeweiches Element zu verstehen, welches über die Fläche verteilte Öffnungen aufweist, durch die die Flüssigkeit austreten kann. Es ist sinnvoll, die Öffnungen mit einem lichten Öffnungsmaß von höchstens 2 mm und besonders bevorzugt von höchstens 1 mm auszubilden.

Die Kombination aus Innenbeutel und Außenbeutel kann in sehr vorteilhafter Weise zum Aufnehmen auch größerer Gewebemengen eingesetzt werden. Der Zulauf ist dazu vorgesehen, Flüssigkeit aufzunehmen, die von der stromaufwärts des Zulaufs angeordneten Pumpe mit einem zumindest leichten Überdruck bereitgestellt wird. Da aus diesem Grund kein Vakuum an dem Ablauf anliegen muss, das auch über den Zulauf an den Morcellator gelangen muss, entfällt die Notwendigkeit, den Gewebeauffangbehälter steif auszubilden.

Der Gewebeauffangbehälter ist insgesamt sehr leicht und kann platzsparend transportiert bzw. gelagert werden. Aufgrund der geringeren Materialmenge und einem kleineren Mindestvolumen im Vergleich zum starr ausgebildeten Gewebeauffangbehälter kann eine höhere Packdichte erreicht werden, was sich je nach Sterilisationsmethode positiv auf die Kosten pro Beutel auswirken kann. Eine größere Anzahl von erfindungsgemäßen Gewebeauffangbehältern als bei bekannten Varianten kann zudem gleichzeitig in einer entsprechenden Sterilisationsvorrichtung aufgenommen und sterilisiert werden. Vorzugsweise kann das Gewebeauffangsystem, bzw. zumindest dessen Gewebeauffangbehälter, als Einwegartikel ausgestaltet sein, welcher nach einmaliger Verwendung entsorgt wird. Eine Sterilisation des Gewebeauffangbehölters wird vorzugsweise werksseitig vorgenommen bzw. wird dieser bereits steril hergestellt und verpackt an den Anwender ausgeliefert, sodass anwenderseitig keine Sterilisation vorgenommen werden muss.

Die Pumpe ist als Teil des Gewebeauffangsystems stromaufwärts des Gewebeauffangbehälters angeordnet, um die betreffende Flüssigkeit druckbeaufschlagt dem Gewebeauffangbehälter zuzuführen. Sie ist bevorzugt dazu in der Lage, an einer von dem Gewebeauffangbehälter abgewandten stromaufwärts gelegenen Seite ein Vakuum bereitzustellen, welches dem Morcellator zuführbar ist. Dort kann auf übliche Weise Gewebe ab- bzw. angesaugt werden. Durch die Anordnung der Pumpe stromaufwärts des Gewebeauffangbehälters ergibt sich die Möglichkeit, diesen biegeweich auszuführen.

Die Pumpe könnte eine Peristaltikpumpe sein. Diese ist auch als Schlauch- oder Schlauchquetschpumpe bekannt und weist einen Schlauch auf, der gezielt mit einer Rolle oder ähnlichem abgeklemmt wird. Durch Bewegen der Rolle wird der Abklemmbereich entlang der Erstreckung des Schlauchs um eine vorgegebene Strecke bewegt. Dadurch entsteht auf der in Bewegungsrichtung folgenden Seite des Schlauchs ein Überdruck, während auf der entgegen der Bewegungsrichtung liegenden Seite ein Unterdruck entsteht.

Der Innenbeutel könnte ein offenmaschiges Gewebe und/oder eine Gitterfolie aufweisen. Es ist vorstellbar, dass der Innenbeutel aus demselben Material besteht wie der Außenbeutel. Durch gezieltes Einbringen einer Perforation oder von Öffnungen könnte die offenmaschige Struktur hergestellt werden. Es ist jedoch auch denkbar, dass der Innenbeutel eine Art Träger aufweist, der an zwei einander gegenüberliegenden Wandungen jeweils eine größere Öffnung aufweist, die mit einer separat hergestellten Gitterfolie oder einem anderen Gewebe abgedeckt sind. Die Verbindung kann durch Schweißen oder Kleben erfolgen. Neben Kunststoffmaterialien ist auch denkbar, die Gitterfolie bzw. das Gewebe durch ein metallisches Material auszubilden.

Der Ablauf könnte an einer Unterseite des Außenbeutels angeordnet sein. Insbesondere könnte der Ablauf am tiefsten Punkt des Außenbeutels angeordnet sein, sodass sich dort schwerkraftbedingt die Flüssigkeit sammelt und aus dem Ablauf treten kann. Der Außenbeutel kann daher zumindest weitgehend restentleert werden.

Der Ablauf könnte von dem Innenbeutel beabstandet sein. Insbesondere kann der Ablauf in vertikaler Richtung von einer Unterkante des Innenbeutels beabstandet sein. Zwischen dem Innenbeutel und dem Außenbeutel ist folglich ein Art Puffer vorgesehen, in dem sich die von dem Innenbeutel in den Außenbeutel tretende Flüssigkeit primär sammelt. Kann die Flüssigkeit während der Nutzung des Gewebeauffangbehälters kontinuierlich aus dem Ablauf abfließen, bleibt der Flüssigkeitspegel in dem Außenbehälter begrenzt. Die in dem Innenbeutel aufgefangenen Gewebepartikel sammeln sich folglich schwerkraftbedingt ebenso zunächst im unteren Bereich des Innenbeutels an. Ein bevorzugt gleichbleibend niedriger Flüssigkeitspegel im Außenbeutel führt dazu, dass die Gewebepartikel im Innenbeutel nicht durch einen zu hohen Flüssigkeitspegel in dem Außenbeutel aufschwimmen und folglich nicht die oberen Öffnungen der offenmaschigen Wandung zusetzen.

Der Außenbeutel könnte weiterhin eine obere Öffnung aufweisen, in die der Zulauf des Innenbeutels ragt. Die Öffnung kann den Zulauf umgeben und dort zu dem Zulauf entsprechend abgedichtet sein. Der Innenbeutel kann im Bereich der oberen Öffnung an dem Außenbeutel befestigt sein, beispielsweise durch eine Schweiß- oder Klebeverbindung. In dieser kann der Zulauf abgedichtet integriert sein.

Der Innenbeutel könnte im Bereich der oberen Öffnung mit dem Außenbeutel verbunden sein. Der Innenbeutel könnte insbesondere ausschließlich mit einem oberen Ende mit dem Bereich der oberen Öffnung verbunden sein. Dieser Bereich kann eine mechanische Schnittstelle zwischen den beiden Beuteln sowie dem Zulauf bilden. Wenn der Innenbeutel nur an dieser Stelle angebunden ist, kann er sich frei bewegen und sich folglich stets so formen bzw. auswölben, wie es aufgrund der Beladung mit Gewebepartikeln oder einer Druckdifferenz über dem Innenbeutel erforderlich ist.

Der Außenbeutel könnte im Bereich der oberen Öffnung einen oberen, starren, plattenförmigen Körper aufweisen, durch den sich der Zulauf erstreckt. Der plattenförmige Körper könnte den Innenbeutel und den Außenbeutel lokal mechanisch stabilisieren, sodass etwa ausgeschlossen wird, dass der Innenbeutel oder der Außenbeutel den Zulauf blockieren. Der Innenbeutel und/oder der Außenbeutel können an Außenkanten des plattenförmigen Körpers angrenzen. Der Zulauf könnte sich auf eine durchgehende Bohrung durch den oberen plattenförmigen Körper beschränken. Dort könnte weiterhin ein Schlauchstutzen angeordnet sein, auf den ein Zulaufschlauch schiebbar ist.

Eine Aufhängevorrichtung, beispielsweise in Form eines Hakens oder einer Öse, könnte an dem Außenbeutel angeordnet sein. Es ist denkbar, dass die Aufhängevorrichtung am oberen Ende des Außenbeutels angeordnet ist. Die Aufhängevorrichtung dient zum Aufhängen des Gewebeauffangbehälters während seiner Benutzung. Bei der Verbindung beider Beutel an dem oberen Ende ist die Platzierung der Aufhängevorrichtung dort besonders sinnvoll.

Der Außenbeutel könnte einen oberen Abschnitt und einen unteren Abschnitt aufweisen, wobei vorgesehen ist, dass der obere Abschnitt eine im Wesentlichen konstante Breite aufweist und den Innenbeutel umschließt, sich der Innenbeutel zu mindestens 90% innerhalb des oberen Abschnitts erstreckt, und der untere Abschnitt eine sich zu dem Ablauf hin verjüngende Breite aufweist. Besonders bevorzugt befindet sich der Innenbeutel ausschließlich in dem oberen Abschnitt. Der untere Abschnitt bildet ein Puffervolumen zwischen dem Innenbeutel und dem Außenbeutel aus. Das Puffervolumen ist durch die sich verjüngende Breite wie ein Trichter geformt, sodass die Flüssigkeit stets in Richtung des Ablaufs geführt wird. Dieser könnte an einer engsten Stelle des unteren Abschnitts angeordnet sein.

Der Außenbeutel könnte im Bereich des Ablaufs unteres Anschlusselement aufweisen, durch das sich der Ablauf erstreckt. Ähnlich wie der vorangehend genannte obere plattenförmige Körper könnte mit dem unteren Anschlusselement eine lokale Stabilisierung des Außenbeutels erreicht werden. Dies verhindert unter anderem den unbeabsichtigten Verschluss des Ablaufs durch eine Falte des Außenbeutels. Der Ablauf könnte als eine durchgehende Bohrung ausgeführt sein, die sich durch das untere Anschlusselement erstreckt. Dort kann ebenso ein Schlauchstutzen angebracht sein. Das untere Anschlusselement lässt sich zum Anschließen eines Schlauchs oder zum Ausfalten des Gewebeauffangbehälters leichter handhaben, da es sich vorzugsweise unter dem Griff eines Benutzers nicht verformt bzw. nachgibt.

Die Erfindung ist nachfolgend anhand von in einer Zeichnung dargestellten Ausführungsbeispiels näher erläutert. Es zeigt:

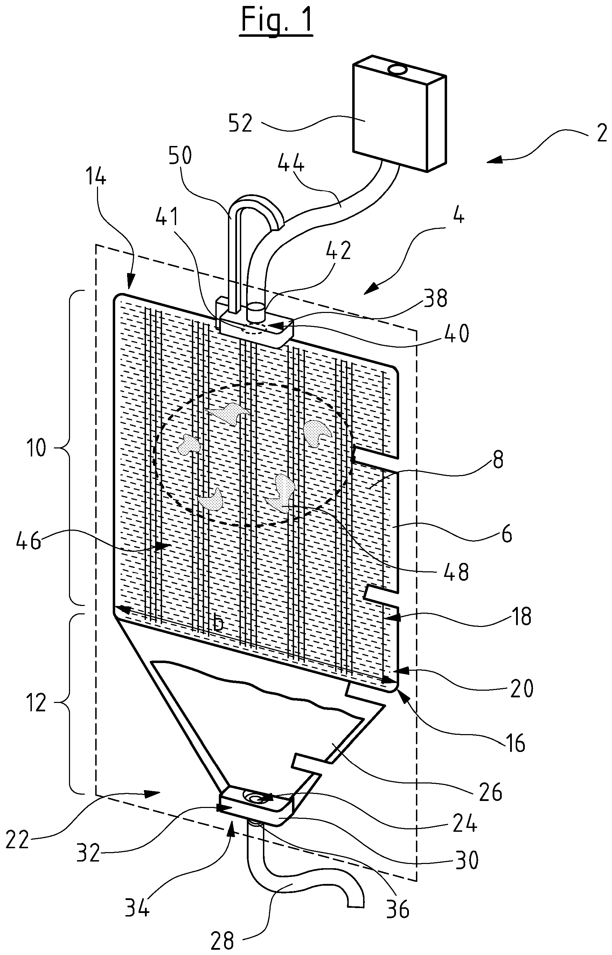

- Fig. 1: eine schematische Ansicht eines Gewebeauffangsystems.

Fig. 1 zeigt ein Gewebeauffangsystem 2, das einen Gewebeauffangbehälter 4 aufweist. Dieser weist einen offenmaschigen Innenbeutel 6 und einen flüssigkeitsdichten Außenbeutel 8 auf, wobei der Außenbeutel 8 den Innenbeutel 6 umschließt. Der Außenbeutel 8 weist einen oberen Abschnitt 10 sowie einen unteren Abschnitt 12 auf. Der Innenbeutel 8 befindet sich beispielhaft ausschließlich in dem oberen Abschnitt 10. Die Breite b des Außenbeutels 6 in dem oberen Abschnitt 10 ist von einer Oberkante 14 bis zu einem Übergangsbereich 16 zwischen dem oberen Abschnitt 10 und dem unteren Abschnitt 12 gleichbleibend. Beide Beutel 6 und 8 bilden damit zumindest in dem oberen Abschnitt 10 eine schlauchartige Anordnung aus. In dem unteren Abschnitt 12 wird die Breite b des Außenbeutels 6 mit zunehmendem Abstand von der Oberkante 14 bzw. dem Übergangsbereich 16 kontinuierlich geringer.

In einem ungefüllten, unbenutzten Zustand bilden die beiden Beutel 6 und 8 eine flache Anordnung aus, deren Dicke weitgehend nur von der Materialstärke der Beutel 6 und 8 abhängt. Beide Beutel 6 und 8 sind aus einem biegeweichen Material hergestellt, sodass sie flach oder zusammengerollt gelagert werden können. Zur Anordnung in dem Außenbeutel 6 weist der Innenbeutel 8 äußere Abmessungen auf, die etwas kleiner dimensioniert sind als die korrespondierenden Innenmaße des Außenbeutels 6. Der Innenbeutel 8 folgt mit seinen Außenkanten 18 jedoch in einem weitgehend gleich bleibenden Abstand Außenkanten 20 des Außenbeutels 6.

An einem unteren Ende 22 des Außenbeutels 6 ist ein Ablauf 24 vorgesehen, durch den eine im Innern des Außenbeutels 6 befindliche Flüssigkeit 26 austreten kann. Der Ablauf 24 ist dafür mit einem Ablaufschlauch 28 verbunden. Weiterhin ist im Bereich des Ablaufs 24 ein unteres Anschlusselement 30 angeordnet und mit dem Außenbeutel 6 verbunden. Das untere Anschlusselement 30 dient hier dem Anschluss des Ablaufschlauchs 28. Es kann entfallen, wenn der Ablaufschlauch 28 direkt mit dem Außenbeutel 6 verbunden ist.

An einer Unterseite 34 des unteren Anschlusselements 30 kann ein unterer Schlauchstutzen 36 angeordnet sein, in den der Abflussschlauch 28 eingesteckt werden kann. Der Ablauf 24 könnte in der beispielhaften Darstellung als eine durchgehende Bohrung durch das Anschlusselement 30 gebildet sein, wobei die Bohrung in dem unteren Schlauchstutzen 36 mündet. Das untere Anschlusselement 30 könnte auch gleich mit einem Durchflusskanal hergestellt sein.

An dem oberen Ende 14 des Innenbeutels 8 ist ein oberes Anschlusselement 38 angeordnet und mit dem Innenbeutel 8 verbunden. Das obere Anschlusselement 38 dient hier dem Anschluss eines Zulaufschlauchs 44. Es kann entfallen, wenn der Zulaufschlauch 44 direkt mit dem Innenbeutel 8 verbunden ist. Der Innenbeutel 8 weist einen Zulauf 40 auf, der sich durch eine obere Öffnung 41 des Außenbeutels 6 in das obere Anschlusselement 38 erstreckt und dort in einen oberen Schlauchstutzen 42 mündet. Der Zulaufschlauch 44 ist an dem oberen Schlauchstutzen 42 angeordnet, über den eine Gewebe enthaltende Flüssigkeit in den Innenbeutel 8 geleitet werden kann. Der Außenbeutel 8 ist rundum geschlossen und weist leidglich die obere Öffnung 41 und den Ablauf 24 als Strömungswege zwischen einer Innenseite und einer Außenseite des Außenbeutels 6 auf. Die Wandungen des Außenbeutels 6 sind flüssigkeitsdicht.

Der Innenbeutel 8 ist rundum geschlossen, wobei ein Innenraum des Innenbeutels 8 mit dem Zulauf 40 in Fluidverbindung steht. Zudem weist der Innenbeutel 8 offenmaschige Wandungen 46 auf, die so feinmaschig sind, dass die Flüssigkeit 26 aus dem Innenbeutel 6 in den Außenbeutel 8 gelangen kann, darin enthaltene Gewebepartikel 48 jedoch zurückgehalten werden. Die aus dem Innenbeutel 8 tretende Flüssigkeit 26 sammelt sich beispielhaft in dem unteren Abschnitt 12 des Außenbeutels 6 und strömt in Richtung des Ablaufs 24. Die durch die kleiner werdende Breite erzielte Trichterform des unteren Abschnitts 12 unterstützt dabei die Entleerung des Außenbeutels 8. Die ausströmende Flüssigkeit wird über den Ablaufschlauch 28 dem Gewebeauffangbehälter 4 entnommen.

Der Gewebeauffangbehälter 4 weist eine Aufhängevorrichtung 50 in Form eines Hakens oder einer Öse auf, die beispielhaft an dem oberen Ende des Außenbeutels 6 angeordnet ist. Hierüber kann der Gewebeauffangbehälter 4 zur Benutzung aufgehängt werden. Der untere Abschnitt 12 des Außenbeutels 6 ist der Aufhängevorrichtung 50 entgegengesetzt angeordnet und der Gewebeauffangbehälter 4 richtet sich beim Aufhängen schwerkraftbedingt in die in Fig. 1 gezeigte Ausrichtung aus.

Stromaufwärts des Zulaufschlauchs 44 ist eine Pumpe 52 angedeutet, die etwa als Peristaltikpumpe ausgeführt ist. Diese kann mit einem Morcellator verbunden sein und stellt kontinuierlich einen Flüssigkeitsstrom mit darin enthaltenen Gewebepartikeln 48 an dem Zulauf 40 bereit. Ein von dem Gewebeauffangbehälter 4 abgewandtes Ende der Pumpe 52 kann mit einem Morcellator (nicht gezeigt) verbunden werden.

### Bezugszeichenliste

- 2: Gewebeauffangsystem
- 4: Gewebeauffangbehälter
- 6: Innenbeutel
- 8: Außenbeutel
- 10: oberer Abschnitt
- 12: unterer Abschnitt
- 14: Oberkante
- 16: Übergangsbereich
- 18: Außenkante Innenbeutel
- 20: Außenkante Außenbeutel
- 22: unteres Ende
- 24: Ablauf
- 26: Flüssigkeit
- 28: Ablaufschlauch
- 30: unteres Anschlusselement
- 32: umlaufender Rand
- 34: Unterseite
- 36: unterer Schlauchstutzen
- 38: obereres Anschlusselement
- 40: Zulauf
- 41: obere Öffnung
- 42: oberer Schlauchstutzen
- 44: Zulaufschlauch
- 46: offenmaschige Wandung
- 48: Gewebepartikel
- 50: Aufhängevorrichtung
- 52: Pumpe

## Patentansprüche

1. Gewebeauffangsystem (2), aufweisend eine Pumpe (52) und einen Gewebeauffangbehälter (4) zum Abtrennen von Gewebepartikeln (48) aus einer Flüssigkeit (26), wobei der Gewebeauffangbehälter (4) einen biegeweichen Innenbeutel (6) aufweist, der einen Zulauf (40) für eine Gewebepartikel (48) aufweisende Flüssigkeit (26) aufweist, wobei der Innenbeutel (6) zumindest bereichsweise eine offenmaschige Wandung (46) aufweist, die dazu ausgebildet ist, Gewebepartikel (48) zu fangen, **dadurch gekennzeichnet, dass** der Gewebeauffangbehälter (4) einen biegeweichen Außenbeutel (8) aufweist, der Innenbeutel (6) schlaff in dem Außenbeutel (8) angeordnet und von diesem umschlossen ist, und der Außenbeutel (8) flüssigkeitsundurchlässig ist und einen Ablauf (24) zum Abführen von Flüssigkeit aufweist, wobei die Pumpe (52) mit dem Zulauf (40) in Fluidverbindung steht und stromaufwärts des Zulaufs (40) angeordnet ist, um Flüssigkeit mit zumindest leichtem Überdruck durch den Zulauf (40) in den Innenbeutel (6) zu drücken.

2. Gewebeauffangsystem (2) nach Anspruch 1, **dadurch gekennzeichnet, dass** die Pumpe (52) eine Peristaltikpumpe ist.

3. Gewebeauffangsystem (2) nach einem der vorhergehenden Ansprüche, **dadurch gekennzeichnet, dass** der Innenbeutel (6) ein offenmaschiges Gewebe und/oder eine Gitterfolie aufweist.

4. Gewebeauffangsystem (2) nach einem der vorhergehenden Ansprüche, **dadurch gekennzeichnet, dass** der Ablauf (24) an einer Unterseite (34) des Außenbeutels (8) angeordnet ist.

5. Gewebeauffangsystem (2) nach einem der vorhergehenden Ansprüche, **dadurch gekennzeichnet, dass** der Ablauf (24) von dem Innenbeutel (6) beabstandet ist.

6. Gewebeauffangsystem (2) nach einem der vorhergehenden Ansprüche, **dadurch gekennzeichnet, dass** der Außenbeutel (8) eine obere Öffnung (41) aufweist, in die der Zulauf (40) des Innenbeutels (6) ragt.

7. Gewebeauffangsystem (2) nach Anspruch 6, **dadurch gekennzeichnet, dass** der Innenbeutel (6) im Bereich der oberen Öffnung (41) mit dem Außenbeutel (8) verbunden ist.

8. Gewebeauffangsystem (2) nach Anspruch 6 oder 7, **dadurch gekennzeichnet, dass** der Außenbeutel (8) im Bereich der oberen Öffnung (41) ein oberes Anschlusselement (38) aufweist, durch das sich der Zulauf (40) erstreckt.

9. Gewebeauffangsystem (2) nach einem der vorhergehenden Ansprüche, **dadurch gekennzeichnet, dass** eine Aufhängevorrichtung (50) an dem Außenbeutel (8) angeordnet ist.

10. Gewebeauffangsystem (2) nach einem der vorhergehenden Ansprüche, **dadurch gekennzeichnet, dass** der Außenbeutel (8) einen oberen Abschnitt (10) und einen unteren Abschnitt (12) aufweist, der obere Abschnitt (10) eine im Wesentlichen konstante Breite (b) aufweist und den Innenbeutel (6) umschließt, sich der Innenbeutel (6) zu mindestens 90% innerhalb des oberen Abschnitts (10) erstreckt, und der untere Abschnitt (12) eine sich zu dem Ablauf (24) hin verjüngende Breite (b) aufweist.

11. Gewebeauffangsystem (2) nach einem der vorhergehenden Ansprüche, **dadurch gekennzeichnet, dass** der Außenbeutel (8) im Bereich des Ablaufs (24) ein unteres Anschlusselement (30) aufweist, durch das sich der Ablauf (24) erstreckt.

## Claims

1. A tissue collecting system (2), having a pump (52) and a tissue collecting container (4) for separating tissue particles (48) from a liquid (26), wherein the tissue collecting container (4) has a flexible inner bag (6), which has an inlet (40) for liquid (26) that contains tissue particles (48), wherein the inner bag (6), at least in some regions, has an open-mesh wall (46) which is designed to catch tissue particles (48), **characterised in that** the tissue collecting container (4) has a flexible outer bag (8), the inner bag (6) is arranged slack in the outer bag (8) and is enclosed by the latter, and the outer bag (8) is impermeable to liquid and has an outlet (24) for the discharging of liquid, wherein the pump (52) is fluidically connected to the inlet (40) and is disposed upstream of the inlet (40) in order to push liquid with at least slight overpressure through the inlet (40) into the inner bag (6).

2. The tissue collecting system (2) according to claim 1, **characterised in that** the pump (52) is a peristaltic pump.

3. The tissue collecting system (2) according to any one of the preceding claims, **characterised in that** the inner bag (6) has an open-mesh fabric and/or a mesh film.

4. The tissue collecting system (2) according to any one of the preceding claims, **characterised in that** the outlet (24) is arranged on an underside (34) of the outer bag (8).

5. The tissue collecting system (2) according to any one of the preceding claims, **characterised in that** the outlet (24) is spaced apart from the inner bag (6).

6. The tissue collecting system (2) according to any one of the preceding claims, **characterised in that** the outer bag (8) has an upper opening (41), into which the inlet (40) of the inner bag (6) projects.

7. The tissue collecting system (2) according to claim 6, **characterised in that** the inner bag (6) is connected to the outer bag (8) in the region of the upper opening (41).

8. The tissue collecting system (2) according to claim 6 or 7, **characterised in that** the outer bag (8), in the region of the upper opening (41), has an upper connection element (38), through which the inlet (40) extends.

9. The tissue collecting system (2) according to any one of the preceding claims, **characterised in that** a hanging device (50) is disposed on the outer bag (8).

10. The tissue collecting system (2) according to any one of the preceding claims, **characterised in that** the outer bag (8) has an upper portion (10) and a lower portion (12), the upper portion (10) has a substantially constant width (b) and encloses the inner bag (6), the inner bag (6) extends at least 90 % within the upper portion (10), and the lower portion (12) has a width (b) that tapers towards the outlet (24).

11. The tissue collecting system (2) according to any one of the preceding claims, **characterised in that** the outer bag (8), in the region of the outlet (24), has a lower connection element (30), through which the outlet (24) extends.

## Revendications

1. Système de collecte de tissus (2), comprenant une pompe (52) et un récipient de collecte de tissus (4) pour séparer les particules de tissu (48) d'un liquide (26), le récipient de collecte de tissus (4) présentant un sac intérieur souple (6), qui a une entrée (40) pour un liquide (26) contenant des particules de tissu (48), le sac intérieur (6) présentant au moins une paroi à mailles ouvertes (46) conçue pour capturer les particules de tissu (48), **caractérisé en ce que** le récipient de collecte de tissus (4) comporte un sac extérieur souple (8), que le sac intérieur (6) est disposé de manière lâche dans le sac extérieur (8) et renfermé par celui-ci, et que le sac extérieur (8) est imperméable au liquide et comporte une évacuation (24) pour évacuer le liquide, la pompe (52) étant en liaison fluidique avec l'entrée (40) et placée en amont de l'entrée (40) pour pousser le liquide dans le sac intérieur (6) avec au moins une légère surpression à travers l'entrée (40).

2. Système de collecte de tissus (2) selon la revendication 1, **caractérisé en ce que** la pompe (52) est une pompe péristaltique.

3. Système de collecte de tissus (2) selon l'une des revendications précédentes, **caractérisé en ce que** le sac intérieur (6) présente un tissu à mailles ouvertes et/ou un film à grille.

4. Système de collecte de tissus (2) selon l'une des revendications précédentes, **caractérisé en ce que** l'évacuation (24) est placée sur une face inférieure (34) du sac extérieur (8).

5. Système collecte de tissus (2) selon l'une des revendications précédentes, **caractérisé en ce que** l'évacuation (24) est espacée du sac intérieur (6).

6. Système de collecte de tissus (2) selon l'une des revendications précédentes, **caractérisé en ce que** le sac extérieur (8) présente une ouverture supérieure (41) dans laquelle l'entrée (40) du sac intérieur (6) dépasse.

7. Système de collecte de tissus (2) selon la revendication 6, **caractérisé en ce que** le sac intérieur (6) est relié au sac extérieur (8) au niveau de l'ouverture supérieure (41).

8. Système de collecte de tissus (2) selon la revendication 6 ou 7, **caractérisé en ce que** le sac extérieur (8) présente, dans la zone de l'ouverture supérieure (41), un élément de raccordement supérieur (38) à travers lequel s'étend l'entrée (40).

9. Système de collecte de tissus (2) selon l'une des revendications précédentes, **caractérisé en ce qu'**un dispositif de suspension (50) est placé sur le sac extérieur (8).

10. Système de collecte de tissus (2) selon l'une des revendications précédentes, **caractérisé en ce que** le sac extérieur (8) comporte une section supérieure (10) et une section inférieure (12), que la section supérieure (10) a une largeur sensiblement constante (b) et enveloppe le sac intérieur (6), que le sac intérieur (6) s'étend pour au moins 90 % à l'intérieur de la section supérieure (10) et que la partie inférieure (12) présente une largeur (b) se rétrécissant en direction de l'évacuation (24).

11. Système de collecte de tissus (2) après l'un des précédents, **caractérisé en ce que** le sac extérieur (8) présente au niveau de l'évacuation (24) un élément de connexion inférieur (30) à travers lequel s'étend l'évacuation (24).
